# EUROPEAN PATENT APPLICATION

(11) **EP 4 847 066 A1**
(43) Date of publication of application: **02.12.2026**
(21) Application number: 25744625.2
(22) Date of filing: 22.01.2025
(51) Int. Cl.: A61M 11/00, H02K 7/06, H02K 7/116

(54) **DRIVING ASSEMBLY AND ELECTRONIC ATOMIZATION DEVICE**

(30) Priority: 24.01.2024 CN 202420187163 U
(71) Applicant: Shenzhen Moore Vaporization Health & Medical Technology Co., Ltd., Shenzhen, Guangdong 518102 (CN)
(72) Inventor: ZHAO, Tao, Shenzhen, Guangdong 518102 (CN); ZHENG, Xin, Shenzhen, Guangdong 518102 (CN); FENG, Ke, Shenzhen, Guangdong 518102 (CN)
(74) Representative: Winter, Brandl - Partnerschaft mbB
(86) International application number: PCT/CN2025/074013
(87) International publication number: WO 2025/157187

(57) **Abstract**

Provided are a drive assembly and an electronic atomization device. The drive assembly provided in the present disclosure includes: a push rod; a threaded rod in threaded connection to the push rod; a reduction gearbox connected to the threaded rod; an electric motor connected to the reduction gearbox and configured to provide rotational power for the threaded rod through the reduction gearbox; and a bearing, where one end of the threaded rod passes through the bearing and is in transmission connection to a gear in the reduction gearbox; and the bearing is in rigid connection to a housing of the reduction gearbox, and an inner ring of the bearing is in fixed connection to the threaded rod. In this way, the one end of the threaded rod is in transmission connection to the gear in the reduction gearbox, the inner ring of the bearing is in fixed connection to the threaded rod, and the bearing is in rigid connection to the housing of the reduction gearbox. Limitation on the threaded rod in the axial direction is increased by the reduction gearbox and the bearing. Moreover, guidance of the threaded rod in the axial direction is strengthened by the bearing. Thus, the end of the threaded rod away from the reduction gearbox can be more stably rotated, and stability of liquid pushing is improved.

## Description

### CROSS-REFERENCE TO RELATED APPLICATIONS

The present disclosure claims the priority to and benefit of Patent Application No. 202420187163.4 filed with the China National Intellectual Property Administration on January 24, 2024, which is incorporated in its entirety herein by reference.

### TECHNICAL FIELD

The present disclosure relates to the technical field of atomization, and particularly relates to a drive assembly and an electronic atomization device.

### BACKGROUND

In the related technology, some electronic atomization devices, such as a facial oxygen injector, an esthetic restoration instrument, and a medical atomizer, atomize an atomization substrate through an atomization element to generate an aerosol. In some solutions, the atomization substrate can be pushed into the atomization element through an active liquid supply method.

A piston-type atomization cartridge can actively supply a liquid to the atomization element. However, an existing drive assembly cannot stably push the liquid, which degrades user experience. Thus, it has vital practical significance to solve the problem that liquid pushing by an existing atomization device is unstable and ineffective.

### SUMMARY

The present disclosure mainly solves the technical problem that liquid pushing by an existing atomization device is unstable and ineffective.

To solve the above technical problem, a technical solution employed by the present disclosure is to provide a drive assembly. The drive assembly includes: a push rod; a threaded rod in threaded connection to the push rod and configured to drive the push rod to move in the axial direction; a reduction gearbox connected to the threaded rod; an electric motor connected to the reduction gearbox and configured to provide rotational power for the threaded rod through the reduction gearbox; and a bearing, where one end of the threaded rod passes through the bearing and is in transmission connection to a gear in the reduction gearbox. The bearing is in rigid connection to a housing of the reduction gearbox. An inner ring of the bearing is in fixed connection to the threaded rod.

In an embodiment, a press plate, where the press plate is in rigid connection to the housing of the reduction gearbox. The bearing is in rigid connection to the press plate. The one end of the threaded rod passes through the bearing and the press plate.

In an embodiment, a groove is provided on the surface of the press plate away from the reduction gearbox. A through hole is provided at the bottom of the groove. The bearing is fixedly arranged in the groove. The one end of the threaded rod passes through the bearing and the through hole.

In an embodiment, the end face of the bearing away from the reduction gearbox is flush with the surface of the press plate away from the reduction gearbox, or the end face of the bearing away from the reduction gearbox is higher than the surface of the press plate away from the reduction gearbox.

In an embodiment, the wall of the through hole and the one end of the threaded rod are spaced apart from each other. A seal member is arranged between the wall of the through hole and the side face of the one end of the threaded rod. The diameter of the through hole is greater than or equal to the outer diameter of the inner ring of the bearing and is less than or equal to the inner diameter of an outer ring of the bearing.

The present disclosure further provides an electronic atomization device. The electronic atomization device includes:
a shell provided with an accommodation cavity;
a liquid storage assembly arranged in the accommodation cavity;
an atomization assembly in communication with the liquid storage assembly; and
a drive assembly arranged on one side of the liquid storage assembly and configured to push an atomization substrate in the liquid storage assembly into the atomization assembly. The drive assembly includes a push rod, a threaded rod, a reduction gearbox, an electric motor, and a bearing. The threaded rod is in threaded connection to the push rod, and configured to drive the push rod to move in the axial direction. The reduction gearbox is connected to the threaded rod. The electric motor is connected to the reduction gearbox, and configured to provide rotational power for the threaded rod through the reduction gearbox. The one end of the threaded rod passes through the bearing and is in transmission connection to a gear in the reduction gearbox. The bearing is in rigid connection to the housing of the reduction gearbox. An inner ring of the bearing is in fixed connection to the threaded rod.

The liquid storage assembly includes a piston. The end of the push rod of the drive assembly away from the electric motor of the drive assembly abuts against the piston.

The push rod and the threaded rod are fixed to each other by a concave-convex structure through overmolding, for the push rod to perform linear motion on the threaded rod.

In an embodiment, in the electronic atomization device provided in the present disclosure, the drive assembly further includes:
a press plate in rigid connection to the housing of the reduction gearbox, where
a bearing is in rigid connection to the press plate, and one end of the threaded rod passes through the bearing and the press plate; and
a guide device, where the guide device is a piston, and the push rod and the threaded rod are fixed to each other by a concave-convex structure through overmolding, for the push rod to perform linear motion on the threaded rod.

In an embodiment, in the electronic atomization device provided in the present disclosure, a groove is provided on the surface of the press plate away from the reduction gearbox. A through hole is provided at the bottom of the groove. The bearing is fixedly arranged in the groove. The one end of the threaded rod passes through the bearing and the through hole.

In some embodiments, in the electronic atomization device provided in the present disclosure, part of the threaded rod passing through the bearing and the press plate accounts for 8% to 15% of the entire threaded rod. Preferably, the part of the threaded rod passing through the bearing and the press plate accounts for 8%, 9%, 10%, 11%, 12%, 13%, 14%, or 15% of the entire threaded rod. Further preferably, the part of the threaded rod passing through the bearing and the press plate accounts for 10% of the entire threaded rod.

In an embodiment, in the electronic atomization device provided in the present disclosure, the end face of the bearing away from the reduction gearbox is flush with the surface of the press plate away from the reduction gearbox, or the end face of the bearing away from the reduction gearbox is higher than the surface of the press plate away from the reduction gearbox.

In an embodiment, in the electronic atomization device provided in the present disclosure, the wall of the through hole and the one end of the threaded rod are spaced apart from each other. A seal member is arranged between the wall of the through hole and the side face of the one end of the threaded rod.

In an embodiment, in the electronic atomization device provided in the present disclosure, the diameter of the through hole is greater than or equal to the outer diameter of the inner ring of the bearing and is less than or equal to the inner diameter of an outer ring of the bearing.

In an embodiment, in the electronic atomization device provided in the present disclosure, an outer ring of the bearing is in fixed connection to the side wall of the groove, and/or the outer ring of the bearing is in fixed connection to the bottom wall of the groove;
the outer side face of the one end of the threaded rod is in fixed connection to the inner side face of the inner ring of the bearing; and/or an annular flange is arranged on the outer side face of the one end of the threaded rod, and the annular flange is in fixed connection to the end face of the inner ring of the bearing away from the reduction gearbox.

In an embodiment, in the electronic atomization device provided in the present disclosure, the housing of the reduction gearbox includes an annular side wall and a top wall, and the press plate is in fixed connection to the top wall by a bolt.

In an embodiment, in the electronic atomization device provided in the present disclosure, a projection of the press plate on the electric motor covers a projection of the annular side wall on the electric motor.

In an embodiment, a projection of the press plate on the electric motor overlaps a projection of the reduction gearbox on the electric motor.

In an embodiment, the diameter of the threaded rod is 2 mm to 4 mm.

In an embodiment, the length of the threaded rod is 24 mm to 48 mm.

In an embodiment, the length of the threaded rod is 2.5 times to 3.5 times the diameter of the threaded rod.

In an embodiment, the outer diameter of the bearing is 4 mm to 8 mm; and/or
the outer diameter of the bearing is 1.5 times to 3 times the diameter of the threaded rod.

In an embodiment, the diameter of the threaded rod is 2 mm to 4 mm. Preferably, the diameter of the threaded rod is optionally 2 mm, 2.1 mm, 2.2 mm, 2.3 mm, 2.4 mm, 2.5 mm, 2.6 mm, 2.7 mm, 2.8 mm, 2.9 mm, 3.0 mm, 3.1 mm, 3.2 mm, 3.3 mm, 3.4 mm, 3.5 mm, 3.6 mm, 3.7 mm, 3.8 mm, 3.9 mm, or 4.0 mm. Further preferably, the diameter of the threaded rod is 2.98 mm.

In some embodiments, the length of the threaded rod is 24 mm to 48 mm. Preferably, the length of the threaded rod is 24 mm, 25 mm, 26 mm, 27 mm, 28 mm, 29 mm, 30 mm, 31 mm, 32 mm, 33 mm, 34 mm, 35 mm, 36 mm, 37 mm, 38 mm, 39 mm, 40 mm, 41 mm, 42 mm, 43 mm, 45 mm, 47 mm, or 48 mm. Further preferably, the length of the threaded rod is 36 mm.

In some embodiments, the length of the threaded rod is 2.5 times to 3.5 times the diameter of the threaded rod. Preferably, the length of the threaded rod is 2.5 times, 2.6 times, 2.7 times, 2.8 times, 2.9 times, 3.0 times, 3.1 times, 3.2 times, 3.3 times, 3.4 times, or 3.5 times the diameter of the threaded rod. Further preferably, in a case that the diameter of the threaded rod is 2.98 mm, the length of the threaded rod is 36 mm.

In some embodiments, the outer diameter of the bearing is 4 mm to 8 mm.

In some embodiments, the outer diameter of the bearing is 1.5 times to 3 times the diameter of the threaded rod.

In an embodiment, the electronic atomization device further includes a gas supply assembly configured to supply gas to the atomization assembly.

In an embodiment, the atomization assembly is a two-phase flow atomization nozzle.

In an embodiment, the liquid storage assembly includes a piston.

In an embodiment, the end of the push rod of the drive assembly away from the electric motor of the drive assembly abuts against the piston.

Beneficial effects of the present disclosure are as follows: the one end of the threaded rod is in transmission connection to the gear in the reduction gearbox, the inner ring of the bearing is in fixed connection to the threaded rod, and the bearing is in rigid connection to the housing of the reduction gearbox. Limitation on the threaded rod in the axial direction is increased by the reduction gearbox and the bearing. Moreover, guidance of the threaded rod in the axial direction is strengthened by the bearing. Thus, the end of the threaded rod away from the reduction gearbox can be more stably rotated, and stability of liquid pushing is improved.

### BRIEF DESCRIPTION OF THE DRAWINGS

FIG. 1 is a structural diagram of an electronic atomization device according to an embodiment of the present disclosure;
FIG. 2 is a schematic structural diagram of a liquid storage assembly according to an embodiment of the present disclosure;
FIG. 3 is an overall schematic diagram of a drive assembly according to an embodiment of the present disclosure;
FIG. 4 is a sectional view in direction A-A in FIG. 3;
FIG. 5 is an overall schematic diagram of a press plate according to an embodiment of the present disclosure;
FIG. 6 is an enlarged view of position P in FIG. 4;
FIG. 7 is an overall schematic diagram of a bearing according to an embodiment of the present disclosure;
FIG. 8 is an enlarged view of position P of a second drive assembly in FIG. 4 according to the present disclosure;
FIG. 9 is a schematic diagram before motion of a push rod of a drive assembly according to the present disclosure;
FIG. 10 is a schematic diagram after motion of a push rod of a drive assembly relative to a threaded rod according to the present disclosure; and
FIG. 11 is a schematic diagram of a position relationship between a push rod and a threaded rod of a drive assembly according to the present disclosure.

Description of reference numerals:
1-electronic atomization device;
2-shell; 3-liquid storage assembly; 31-piston; 4-atomization assembly;
5-drive assembly; 51-push rod; 52-threaded rod; 521-annular flange; 53-reduction gearbox; 530-housing; 531-annular side wall; 532-top wall; 54-electric motor; 55-bearing; 551-inner ring; 552-outer ring; 56-press plate; 561-groove; 562-through hole; 57-seal member; 58-concave-convex structure; and
6-gas supply assembly.

### DETAILED DESCRIPTION

The technical solutions of the embodiments of the present disclosure are clearly and completely described below in combination with the accompanying drawings in the embodiments of the present disclosure. Apparently, the described embodiments are merely some embodiments rather than all embodiments of the present disclosure. Based on the embodiments of the present disclosure, all other embodiments obtained by those of ordinary skill in the art without making creative efforts fall within the scope of protection of the present disclosure.

The terms "first", "second", and "third" in the present disclosure are merely used for the purpose of description, and cannot be construed as indicating or implying relative importance or implying a quantity of indicated technical features. Thus, a feature defined with "first", "second", or "third" can explicitly or implicitly include at least one of the features. In the description of the present disclosure, "a plurality of" means at least two, such as two and three unless it is specifically defined otherwise. All directional indications (such as, up, down, left, right, front, and back...) in the embodiments of the present disclosure are only used for explaining relative position relationships, movement situations, etc. between various components in a particular posture (as shown in the accompanying drawings). If the particular posture changes, the directional indications change accordingly. In addition, the terms "comprise", "include", "have", and any variant thereof are intended to cover non-exclusive inclusion. For example, processes, methods, systems, products, or apparatuses that include a series of steps or units are not limited to the listed steps or units, but optionally further include steps or units that are not listed, or optionally further include other steps or units that are intrinsic to these processes, methods, products, or apparatuses.

Reference to an "embodiment" mentioned herein means that a particular feature, structure, or characteristic described in combination with the embodiment can be encompassed in at least one embodiment of the present disclosure. The phrase appearing in various positions in the description does not necessarily refer to the same embodiment, nor does it refer to an independent or alternative embodiment that is mutually exclusive with another embodiment. Those skilled in the art understand explicitly or implicitly that the embodiment described herein may be combined with other embodiments.

In a related technology, a stepper electric motor drives, through a reduction gearbox, a threaded rod to rotate. The threaded rod and a gear of the reduction gearbox are directly welded together. That is, one end of the threaded rod is fixed first. The other end, that is, a top end, of the threaded rod is likely to deviate during rotation, causing a problem of unstable overall liquid pushing.

In addition, a push rod is arranged at the top end of the threaded rod. The push rod is required to push a piston of an atomization cartridge to move, and the push rod is subjected to a force of 100 N. Through force analysis, a joint between the threaded rod and the reduction gearbox is also subjected to extremely large pressure. In an existing solution, the threaded rod is likely to press down the gear in the reduction gearbox and then the gear is in contact with other components, causing friction of the end face of the gear. Consequently, an overall pushing force is reduced, and abnormal friction noise is generated.

A guide device, where the guide device is a piston. The push rod and the threaded rod are fixed to each other by a concave-convex structure through overmolding, for the push rod to perform linear motion on the threaded rod. The push rod cooperates with a main machine support such that the push rod cannot rotate. The push rod and the threaded rod are fixed to each other by a concave-convex structure through overmolding, such that the push rod and the threaded rod are connected to each other and fixed, to change rotation of the electric motor to linear motion.

In a particular embodiment, the diameter of the threaded rod is precisely designed, and set within a range of 2 mm to 4 mm. Within the range, the diameter of the threaded rod has a plurality of optional values, such as 2 mm, 2.1 mm, 2.2 mm, 2.3 mm, 2.4 mm, 2.5 mm, 2.6 mm, 2.7 mm, 2.8 mm, 2.9 mm, 3.0 mm, 3.1 mm, 3.2 mm, 3.3 mm, 3.4 mm, 3.5 mm, 3.6 mm, 3.7 mm, 3.8 mm, 3.9 mm, and 4.0 mm. Further preferably, in a particular embodiment, the diameter of the threaded rod is 2.98 mm.

In a particular embodiment, the length of the threaded rod is precisely designed, and set within a range of 24 mm to 48 mm. Within the range, the length of the threaded rod has a plurality of optional values, such as 24 mm, 25 mm, 26 mm, 27 mm, 28 mm, 29 mm, 30 mm, 31 mm, 32 mm, 33 mm, 34 mm, 35 mm, 36 mm, 37 mm, 38 mm, 39 mm, 40 mm, 41 mm, 42 mm, 43 mm, 45 mm, 47 mm, and 48 mm. Further preferably, in a particular embodiment, the length of the threaded rod is 36 mm.

In some embodiments, the length of the threaded rod is 2.5 times to 3.5 times the diameter of the threaded rod. Preferably, the length of the threaded rod is 2.5 times, 2.6 times, 2.7 times, 2.8 times, 2.9 times, 3.0 times, 3.1 times, 3.2 times, 3.3 times, 3.4 times, or 3.5 times the diameter of the threaded rod. Further preferably, in a case that the diameter of the threaded rod is 2.98 mm, the length of the threaded rod is 36 mm.

In some embodiments, the outer diameter of the bearing is 4 mm to 8 mm, and the outer diameter of the bearing is 1.5 times to 3 times the diameter of the threaded rod.

In some embodiments, part of the threaded rod passing through the bearing and the press plate accounts for 8% to 15% of the entire threaded rod. Preferably, the part of the threaded rod passing through the bearing and the press plate accounts for 8%, 9%, 10%, 11%, 12%, 13%, 14%, or 15% of the entire threaded rod. Further preferably, in a particular embodiment, the part of the threaded rod passing through the bearing and the press plate accounts for 10% of the entire threaded rod.

Based on this, the embodiments of the present disclosure provide an electronic atomization device, which effectively solves the problems of unstable liquid pushing, decreased pushing force, and abnormal friction noise.

The present disclosure is described in detail below in combination with the accompanying drawings and the embodiments.

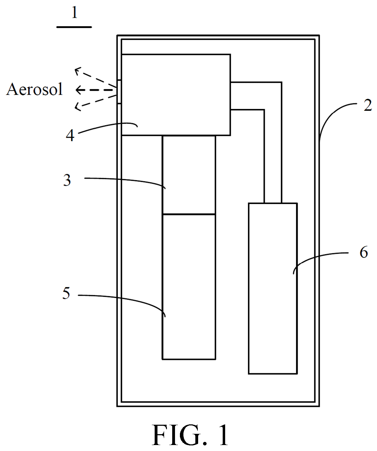

With reference to FIG. 1, a structural diagram of an electronic atomization device according to an embodiment of the present disclosure is shown.

The embodiments of the present disclosure provide an electronic atomization device 1. The electronic atomization device 1 includes a shell 2, a liquid storage assembly 3, an atomization assembly 4, and a drive assembly 5.

In FIG. 1, the shell 2 is provided with an accommodation cavity, and the liquid storage assembly 3 is arranged in the accommodation cavity. The liquid storage assembly 3 is configured to store an atomization substrate. The atomization substrate includes, but is not limited to, a medicinal liquid, a nutrient solution, a cosmetic liquid, an essential liquid, an essential lotion, a disinfectant, an insecticide, a pesticide, etc. Thus, the electronic atomization device 1 to which the atomization substrate is applied can be applied to different fields, such as the medical field, the cosmetic field, and the agricultural field.

The atomization assembly 4 is arranged on one side of the liquid storage assembly 3, and in communication with the liquid storage assembly 3. The liquid storage assembly 3 delivers the atomization substrate to the atomization assembly 4. The drive assembly 5 is arranged on the other side of the liquid storage assembly 3. The drive assembly 5 is configured to push the atomization substrate in the liquid storage assembly 3 into the atomization assembly 4. After the atomization assembly 4 atomizes the atomization substrate, an aerosol is formed to be used by a user.

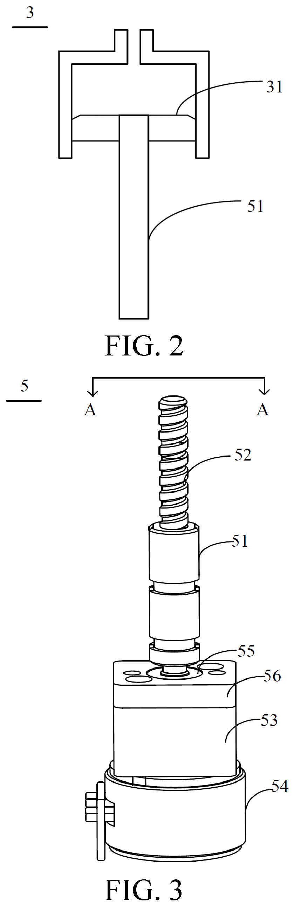

As shown in FIG. 2, a schematic structural diagram of a liquid storage assembly according to an embodiment of the present disclosure is shown in FIG. 2. In an embodiment, the liquid storage assembly 3 includes a piston 31, and the end of the push rod 51 away from the electric motor 54 abuts against the piston 31. The drive assembly 5 drives the piston 31 through the push rod 51, and the piston 31 pushes, in the liquid storage assembly 3, the atomization substrate to enter the atomization assembly 4.

The atomization assembly 4 may be an ultrasonic atomization assembly, an electric heating atomization assembly, a two-phase flow atomization nozzle, etc. in the prior art. In the embodiment, an example in which the atomization assembly 4 is the two-phase flow atomization nozzle is used for description. The electronic atomization device 1 further includes a gas supply assembly 6, and the gas supply assembly 6 is configured to supply gas to the atomization assembly 4. A high-pressure gas can shear the atomization substrate entering the atomization assembly 4 into atomization particles having a suitable particle size, that is, an aerosol, and carry the aerosol out of a spraying port of the atomization assembly 4. The gas supply assembly 6 may include a gas pump.

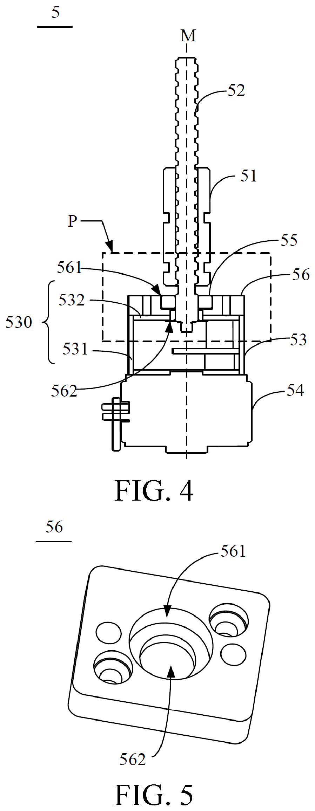

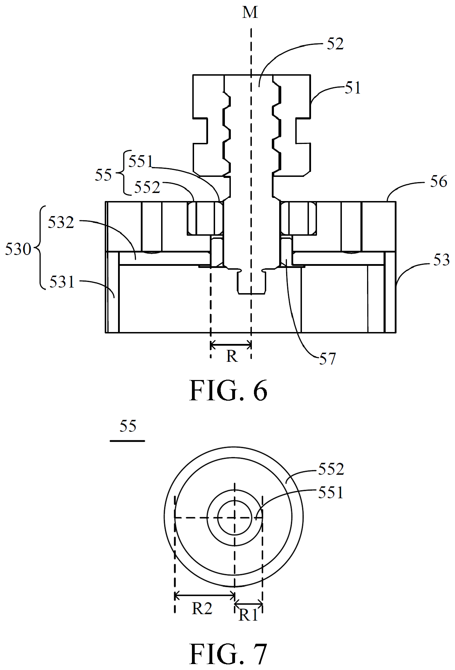

FIG. 3 is an overall schematic diagram of a drive assembly according to an embodiment of the present disclosure. FIG. 4 is a sectional view in direction A-A in FIG. 3. FIG. 5 is an overall schematic diagram of a press plate according to an embodiment of the present disclosure. FIG. 6 is an enlarged view of position P in FIG. 4. FIG. 7 is an overall schematic diagram of a bearing according to an embodiment of the present disclosure.

In an embodiment, the shape of the press plate is a cuboid. Two screw holes are diagonally provided in the middle, and a hole for placing a bearing is also provided in the middle.

In an embodiment, as shown in FIG. 3, the drive assembly 5 includes a push rod 51, a threaded rod 52, a reduction gearbox 53, an electric motor 54, and a bearing 55.

The threaded rod 52 is in threaded connection to the push rod 51, and the threaded rod 52 is configured to drive the push rod 51 to move in the axial direction. The end of the push rod 51 away from the electric motor 54 abuts against the piston 31, and the push rod 51 is configured to push the piston 31 to move. The reduction gearbox 53 is connected to the threaded rod 52. The electric motor 54 is connected to the reduction gearbox 53. The electric motor 54 is configured to provide rotational power for the threaded rod 52 through the reduction gearbox 53. The threaded rod 52 drives the push rod 51 to move. The push rod 51 pushes the piston 31 in the liquid storage assembly 3 such that the atomization substrate in the liquid storage assembly 3 can be delivered to the atomization assembly 4. After the atomization substrate is atomized, an aerosol is formed to be used by a user.

With reference to FIG. 4, the end of the threaded rod 52 close to the electric motor 54 passes through the bearing 55, and is in transmission connection to a gear (not shown in the figure) in the reduction gearbox 53. The bearing 55 is in rigid connection to a housing 530 of the reduction gearbox 53. The bearing 55 may be directly in rigid connection to the housing 530 of the reduction gearbox 53. For example, the bearing 55 may be in fixed connection to the housing 530 of the reduction gearbox 53 through welding, or serve as a part of the housing 530 of the reduction gearbox 53. Or, the bearing 55 may be indirectly in rigid connection to the reduction gearbox 53 through another connector, such as a press plate 56. With reference to FIG. 6, an inner ring 551 of the bearing 55 is in fixed connection to the threaded rod 52, for example, through welding and interference fitting.

In this way, the one end of the threaded rod 52 is in transmission connection to the gear (not shown in the figure) in the reduction gearbox 53. The inner ring 551 of the bearing 55 is in fixed connection to the threaded rod 52. The bearing 55 is in rigid connection to the housing 530 of the reduction gearbox 53. Limitation on the threaded rod 52 in the axial direction is increased by the reduction gearbox 53 and the bearing 55. Moreover, guidance of the threaded rod 52 in the axial direction is strengthened through constraint by the bearing 55 on the threaded rod 52. Thus, the end of the threaded rod 52 away from the reduction gearbox 53 can be more stably rotated, and stability of liquid pushing is improved.

It can be understood that the bearing 55 is in rigid connection to the housing 530 of the reduction gearbox 53, and the inner ring 551 of the bearing 55 is in fixed connection to the threaded rod 52. Thus, the threaded rod 52 is also in rigid connection to the housing 530 of the reduction gearbox 53. In this way, in an operating process of the drive assembly 5, the electric motor 54 drives the reduction gearbox 53 to operate, the reduction gearbox 53 drives the threaded rod 52 to rotate, the threaded rod 52 moves the push rod 51 in the axial direction, and the push rod 51 pushes the piston 31 to move in the axial direction. In a moving process of the piston 31, it can be seen through force analysis that the push rod 51 is simultaneously subjected to resistance in the axial direction, and the threaded rod 52 is also subjected to an extremely large action force. Since the threaded rod 52 is in fixed connection to the bearing 55 and the bearing 55 is in rigid connection to the housing 530 of the reduction gearbox 53, an action force exerted to the threaded rod 52 is transmitted to the shell of the reduction gearbox 53 through the bearing 55. In this way, the gear (not shown in the figure) in the reduction gearbox 53 is no longer affected by pressure of the threaded rod 52 in the axial direction. Moreover, an action force exerted by the threaded rod 52 does not act on the electric motor 54. Thus, an original upper limit of a force exerted to the electric motor 54 is increased, and service life of the electric motor 54 subjected to a force is prolonged.

In an embodiment, as shown in FIG. 3 and FIG. 4, the drive assembly 5 further includes a press plate 56. The press plate 56 is in rigid connection to the housing 530 of the reduction gearbox 53. The bearing 55 is in rigid connection to the press plate 56. The press plate 56 is provided with an opening for the threaded rod 52 to pass through. One end of the threaded rod 52 passes through the bearing 55 and the press plate 56, and is in transmission connection to the gear (not shown in the figures) in the reduction gearbox 53. In this way, the press plate 56 is arranged, the press plate 56 is in rigid connection to the housing 530 of the reduction gearbox 53, the bearing 55 is in rigid connection to the press plate 56, and the inner ring 551 of the bearing 55 is in fixed connection to the threaded rod 52. Thus, limitation on the threaded rod 52 in the axial direction is increased by the reduction gearbox 53 and the bearing 55. Moreover, the bearing 55 can be favorably in rigid connection to the housing 530 of the reduction gearbox through the press plate 56. A mounting process is simple.

Specifically, in an embodiment, as shown in FIG. 4 and FIG. 5, the press plate 56 is a plate-shaped body having strength, and may be a metal plate such as a steel plate. A groove 561 is provided on the surface of the press plate 56 away from the reduction gearbox 53. A space of the groove 561 may be a cylinder, a prism, etc., and the space of the groove 561 is sufficient to accommodate the bearing 55. A through hole 562 is provided at the bottom of the groove 561, and the shape of the through hole 562 may be a square, a circle, etc. The bearing 55 is fixedly arranged in the groove 561. The bearing 55 and the through hole 562 of the groove 561 are coaxially arranged in the central axis M. One end of the threaded rod 52 passes through the bearing 55 and the through hole 562, and is in transmission connection to the gear (not shown in the figures) in the reduction gearbox 53.

Specifically, in an embodiment, as shown in FIG. 5, the groove is a frustum-of-cone surface, and the length of the groove extends through the press plate. The diameter of the large circle is equal to the diameter of the bearing and is 6 mm, and the diameter of the small circle is about 3 mm.

In an embodiment, as shown in FIG. 6, the end face of the bearing 55 away from the reduction gearbox 53 is flush with the surface of the press plate 56 away from the reduction gearbox 53. In this way, the end face of the bearing 55 away from the reduction gearbox 53 does not protrude from the surface of the press plate 56 away from the reduction gearbox 53. The overall size of the press plate 56 is not increased such that mounting of other components cannot affected. Or, the end face of the bearing 55 away from the reduction gearbox 53 is higher than the surface of the press plate 56 away from the reduction gearbox 53. In this way, the contact area between the bearing 55 and the threaded rod 52 in the axial direction is increased. Thus, constraint by the bearing 55 on the threaded rod 52 is strengthened, the threaded rod 52 can be better prevented from being inclined, and the threaded rod 52 can be more stably rotated.

In an embodiment, as shown in FIG. 4 and FIG. 6, the housing 530 of the reduction gearbox 53 includes an annular side wall 531 and a top wall 532. The press plate 56 is in fixed connection to the top wall 532 by a bolt (not shown in the figures). A projection of the press plate 56 on the electric motor 54 covers a projection of the annular side wall 531 on the electric motor 54. In this way, the press plate 56 can be subjected to a supporting force from the annular side wall 531 and the top wall 532 of the reduction gearbox 53.

In an embodiment, a projection of the press plate 56 on the electric motor 54 overlaps a projection of the reduction gearbox 53 on the electric motor 54. In this way, the size of the reduction gearbox 53 in the radial direction is not increased. That is, only the length of the reduction gearbox 53 in the axial direction is increased. Thus, mounting of other components, such as a sensor and a keyboard plate, cannot be affected.

In an embodiment, as shown in FIG. 6, the wall of the through hole 562 is spaced apart from the one end of the threaded rod 52. In this way, the threaded rod 52 and the through hole 562 are spaced apart from each other. An electric motor 54 drives, through the reduction gearbox 53, the threaded rod 52 to rotate. During rotation, the threaded rod 52 is not in contact with the wall of the through hole 562 such that rotation of the threaded rod 52 cannot be affected. In an embodiment, a seal member 57 is arranged between the wall of the through hole 562 and the side face of the one end of the threaded rod 52. The seal member 57 is an annular seal member 57. The material of the seal member 57 is silicone. The seal member 57 is configured to block a gap between the through hole 562 and the threaded rod 52. Thus, the situation that components are damaged because the atomization substrate is leaked into the reduction gearbox 53 or the electric motor 54 can be prevented. Or a through hole (not marked in the figure) for the threaded rod 52 to be inserted into is provided on the top wall 532 of the reduction gearbox 53 The end of the seal member 57 close to the reduction gearbox 53 extends through the through hole of the top wall 532 of the reduction gearbox 53. The seal member 57 extends to the surface of the top wall 532 close to the electric motor 54, to better seal a gap between the through hole 562 and the threaded rod 52. Thus, the situation that components are damaged because the atomization substrate is leaked into the reduction gearbox 53 or the electric motor 54 can be prevented.

As shown in FIG. 7, the diameter R of the through hole 562 is greater than or equal to the outer diameter R1 of the inner ring 551 of the bearing 55, and is less than or equal to the inner diameter R2 of an outer ring 552 of the bearing 55. In this way, the bearing 55 is fixedly arranged in the groove 561. The bearing 55 and the through hole 562 of the groove 561 are coaxially arranged in the central axis M. The threaded rod 52 can pass through the bearing 55 and the through hole 562 without obstacles. The end of the inner ring 551 of the bearing 55 close to the reduction gearbox 53 is not in contact with the bottom of the groove 561 and is suspended. Thus, the inner ring 551 of the bearing 55 can rotate without interference from the bottom of the groove 561. Moreover, the through hole 562 and the threaded rod 52 are spaced apart from each other such that the seal member 57 can be arranged between the through hole 562 and the threaded rod 52, to seal the gap between the through hole and the threaded rod. The diameter of the through hole 562 should not be too large. It can be understood that the bearing 55 is fixedly arranged in the groove 561. The contact area between the bearing 55 and the bottom wall of the groove 561 should not be too small. Thus, a supporting force by the groove 561 on the bearing 55 can be improved.

In an embodiment, as shown in FIG. 6, the outer ring 552 of the bearing 55 is in fixed connection to the side wall of the groove 561, and/or the outer ring 552 of the bearing 55 is in fixed connection to the bottom wall of the groove 561. In this way, the bearing 55 is in fixed connection to the groove 561 of the press plate 56, such that the bearing 55 is in rigid connection to the press plate 56, and the press plate 56 is in rigid connection to the housing 530 of the reduction gearbox 53. It can be understood that the bearing 55 is in rigid connection to the housing 530 of the reduction gearbox 53. Limitation on the threaded rod 52 in the axial direction is increased by the bearing 55. Moreover, guidance of the threaded rod 52 in the axial direction is strengthened through constraint by the bearing 55 on the threaded rod 52. Thus, the end of the threaded rod 52 away from the reduction gearbox 53 can be more stably rotated, and stability of liquid pushing is improved.

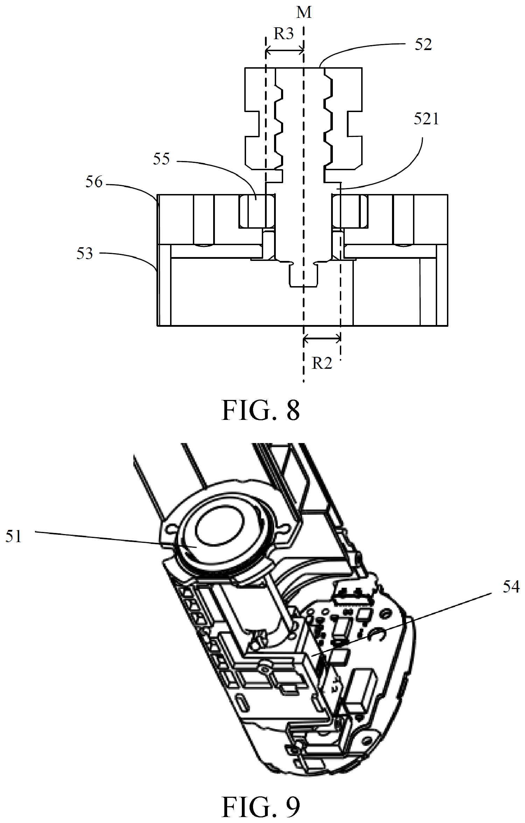

In an embodiment, the outer side face of the one end of the threaded rod 52 is in fixed connection to the inner side face of the inner ring 551 of the bearing 55. In another embodiment, as shown in FIG. 8, an enlarged view of position P of a second drive assembly in FIG. 4 according to the present disclosure is shown in FIG. 8. And/or an annular flange 521 is arranged on the outer side face of the one end of the threaded rod 52. The outer diameter R3 of the annular flange 521 is less than or equal to the outer diameter R2 of the inner ring 551 of the bearing 55. The annular flange 521 is in fixed connection to the end face of the inner ring 551 of the bearing 55 away from the reduction gearbox 53. A supporting force on the bearing 55 in the axial direction is increased by the annular flange 521. The threaded rod 52 is in fixed connection to the bearing 55. The bearing 55 is in rigid connection to the housing 530 of the reduction gearbox 53. An action force exerted to the threaded rod 52 is transmitted to the shell of the reduction gearbox 53 through the bearing 55. In this way, a rotary gear (not shown in the figure) in the reduction gearbox 53 is no longer affected by pressure of the threaded rod 52 in the axial direction. Moreover, an action force exerted by the threaded rod 52 does not act on the electric motor 54. Thus, an original upper limit of a force exerted to the electric motor 54 is increased, and service life of the electric motor 54 subjected to a force is prolonged. Fixed connections in the above embodiments can all be implemented through welding, interference fitting, etc.

The embodiments of the present disclosure provide an electronic atomization device including a drive assembly 5. The drive assembly 5 includes a push rod 51, a threaded rod 52, a reduction gearbox 53, an electric motor 54, and a bearing 55. The threaded rod 52 is in threaded connection to the push rod 51, and configured to drive the push rod 51 to move in the axial direction. The reduction gearbox 53 is connected to the threaded rod 52. The electric motor 54 is connected to the reduction gearbox 53, and configured to provide rotational power for the threaded rod 52 through the reduction gearbox 53. One end of the threaded rod 52 passes through the bearing 55 and is in transmission connection to the gear (not shown in the figure) in the reduction gearbox 53. The bearing 55 is in rigid connection to the housing 530 of the reduction gearbox 53, and an inner ring 551 of the bearing 55 is in fixed connection to the threaded rod 52. In this way, the one end of the threaded rod 52 is in transmission connection to the gear (not shown in the figure) in the reduction gearbox 53. The inner ring 551 of the bearing 55 is in fixed connection to the threaded rod 52. The bearing 55 is in rigid connection to the housing 530 of the reduction gearbox 53. Limitation on the threaded rod 52 in the axial direction is increased by the reduction gearbox 53 and the bearing 55. Moreover, guidance of the threaded rod in the axial direction 52 is strengthened by the bearing 55. Thus, the end of the threaded rod 52 away from the reduction gearbox 53 can be more stably rotated, and stability of liquid pushing is improved.

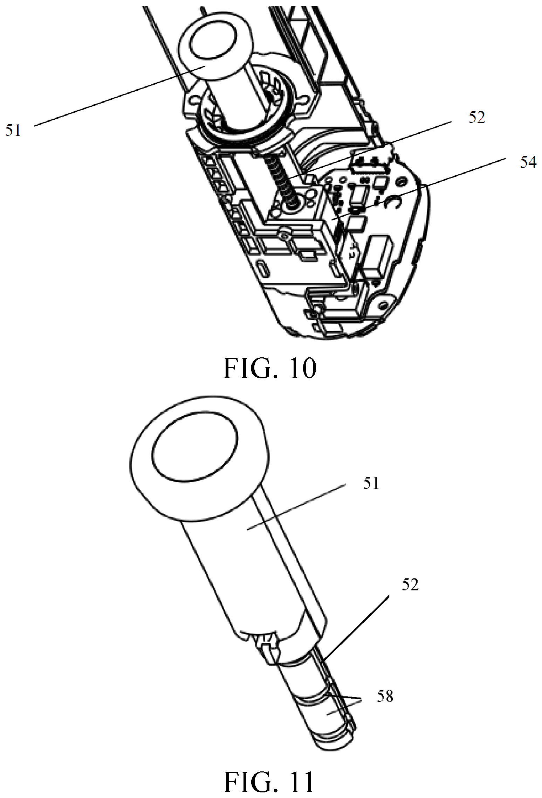

In a particular embodiment, as shown in FIG. 9 to FIG. 11, schematic diagrams of position relationships between a push rod 51 and a threaded rod 52 of a drive assembly according to the present disclosure are shown. FIG. 9 is a schematic diagram before motion of a push rod 51. FIG. 10 is a schematic diagram after motion of a push rod 51 relative to a threaded rod 52. FIG. 11 is a schematic diagram of a position relationship between a push rod 51 and a threaded rod 52 of a drive assembly according to the present disclosure. It can be seen from FIG. 11 that the push rod 51 and the threaded rod 52 are fixed by a concave-convex structure 58. More specifically, in the embodiment, the push rod 51 and the threaded rod 52 are fixed by a concave-convex structure 58 through overmolding, for the push rod 51 to perform linear motion on the threaded rod 52 (as shown in FIG. 9 and FIG. 10). The push rod 51 cooperates with a main machine support of the electronic atomization device such that the push rod cannot rotate. The push rod 51 and the threaded rod 52 are fixed by the concave-convex structure 58 through overmolding. Thus, the push rod 51 and the threaded rod 52 are connected to each other and fixed, to change rotation of the electric motor 54 to linear motion.

The foregoing are merely implementations of the present disclosure, and do not limit the patent scope of the present disclosure. Equivalent structures or equivalent process transformation made by using the description and the accompanying drawings of the present disclosure, or directly or indirectly applied in other related technical fields, are also included in the patent protection scope of the present disclosure.

## Claims

1. A drive assembly, comprising:
a push rod;
a threaded rod in threaded connection to the push rod and configured to drive the push rod to move in the axial direction;
a reduction gearbox connected to the threaded rod;
an electric motor connected to the reduction gearbox and configured to provide rotational power for the threaded rod through the reduction gearbox; and
a bearing, wherein one end of the threaded rod passes through the bearing and is in transmission connection to a gear in the reduction gearbox; and
the bearing is in rigid connection to a housing of the reduction gearbox, and an inner ring of the bearing is in fixed connection to the threaded rod.

2. The drive assembly of claim 1, further comprising:
a press plate in rigid connection to the housing of the reduction gearbox, wherein
the bearing is in rigid connection to the press plate, and the one end of the threaded rod passes through the bearing and the press plate.

3. The drive assembly of claim 2, wherein
a groove is provided on the surface of the press plate away from the reduction gearbox, a through hole is provided at the bottom of the groove, the bearing is fixedly arranged in the groove, and the one end of the threaded rod passes through the bearing and the through hole.

4. The drive assembly of claim 3, wherein
the end face of the bearing away from the reduction gearbox is flush with the surface of the press plate away from the reduction gearbox, or the end face of the bearing away from the reduction gearbox is higher than the surface of the press plate away from the reduction gearbox.

5. The drive assembly of claim 3, wherein
the wall of the through hole and the one end of the threaded rod are spaced apart from each other, and a seal member is arranged between the wall of the through hole and the side face of the one end of the threaded rod; and
the diameter of the through hole is greater than or equal to the outer diameter of the inner ring of the bearing and is less than or equal to the inner diameter of an outer ring of the bearing.

6. An electronic atomization device, comprising:
a shell provided with an accommodation cavity;
a liquid storage assembly arranged in the accommodation cavity;
an atomization assembly in communication with the liquid storage assembly; and
a drive assembly arranged on one side of the liquid storage assembly and configured to push an atomization substrate in the liquid storage assembly into the atomization assembly, wherein the drive assembly comprises a push rod, a threaded rod, a reduction gearbox, an electric motor, and a bearing; the threaded rod is in threaded connection to the push rod, and configured to drive the push rod to move in the axial direction; the reduction gearbox is connected to the threaded rod; the electric motor is connected to the reduction gearbox, and configured to provide rotational power for the threaded rod through the reduction gearbox; one end of the threaded rod passes through the bearing and is in transmission connection to a gear in the reduction gearbox; and the bearing is in rigid connection to the housing of the reduction gearbox, and an inner ring of the bearing is in fixed connection to the threaded rod.

7. The electronic atomization device of claim 6, wherein the liquid storage assembly comprises a piston, and the end of the push rod of the drive assembly away from the electric motor of the drive assembly abuts against the piston.

8. The electronic atomization device of claim 6, wherein the push rod and the threaded rod are fixed to each other by a concave-convex structure, for the push rod to perform linear motion on the threaded rod.

9. The electronic atomization device of claim 6, wherein the drive assembly further comprises:
a press plate in rigid connection to the housing of the reduction gearbox, wherein
the bearing is in rigid connection to the press plate, and one end of the threaded rod passes through the bearing and the press plate.

10. The electronic atomization device of claim 9, wherein part of the threaded rod passing through the bearing and the press plate accounts for 8% to 15% of the entire threaded rod.

11. The electronic atomization device of claim 9, wherein
a groove is provided on the surface of the press plate away from the reduction gearbox, a through hole is provided at the bottom of the groove, the bearing is fixedly arranged in the groove, and one end of the threaded rod passes through the bearing and the through hole.

12. The electronic atomization device of claim 9, wherein
the end face of the bearing away from the reduction gearbox is flush with the surface of the press plate away from the reduction gearbox, or the end face of the bearing away from the reduction gearbox is higher than the surface of the press plate away from the reduction gearbox.

13. The electronic atomization device of claim 11, wherein
the wall of the through hole and the one end of the threaded rod are spaced apart from each other, and a seal member is arranged between the wall of the through hole and the side face of the one end of the threaded rod; and
the diameter of the through hole is greater than or equal to the outer diameter of the inner ring of the bearing and is less than or equal to the inner diameter of an outer ring of the bearing.

14. The electronic atomization device of claim 11, wherein
an outer ring of the bearing is in fixed connection to the side wall of the groove, and/or the outer ring of the bearing is in fixed connection to the bottom wall of the groove;
the outer side face of the one end of the threaded rod is in fixed connection to the inner side face of the inner ring of the bearing; and/or an annular flange is arranged on the outer side face of the one end of the threaded rod, and the annular flange is in fixed connection to the end face of the inner ring of the bearing away from the reduction gearbox.

15. The electronic atomization device of any one of claims 6 to 14, wherein
the housing of the reduction gearbox comprises an annular side wall and a top wall, and the press plate is in fixed connection to the top wall by a bolt; and a projection of the press plate on the electric motor covers a projection of the annular side wall on the electric motor.

16. The electronic atomization device of claim 9, wherein
a projection of the press plate on the electric motor overlaps a projection of the reduction gearbox on the electric motor.

17. The electronic atomization device of claim 6, wherein in the drive assembly,
the diameter of the threaded rod is 2 mm to 4 mm, the length of the threaded rod is 24 mm to 48 mm, and the length of the threaded rod is 2.5 times to 3.5 times the diameter of the threaded rod;
the outer diameter of the bearing is 4 mm to 8 mm; and/or
the outer diameter of the bearing is 1.5 times to 3 times the diameter of the threaded rod.

18. The electronic atomization device of claim 6, further comprising:
a gas supply assembly configured to supply gas to the atomization assembly; and
the atomization assembly is a two-phase flow atomization nozzle.
